# EUROPÄISCHE PATENTSCHRIFT

(11) **EP 2 490 631 B1**
(45) Veröffentlichungstag und Bekanntmachung des Hinweises auf die Patenterteilung: **23.10.2013**
(21) Anmeldenummer: 10765938.5
(22) Anmeldetag: 09.09.2010
(51) Int. Cl.: A61F 5/02, A61F 5/37

(54) **VORRICHTUNG ZUR ERFASSUNG UND/ODER BEEINFLUSSUNG DER KÖRPERHALTUNG**
DEVICE FOR DETECTING AND/OR INFLUENCING POSTURE
DISPOSITIF POUR DÉTECTER ET/OU INFLUENCER LA POSTURE

(30) Priorität: 22.10.2009 DE 102009050385
(43) Veröffentlichungstag der Anmeldung: 29.08.2012
(73) Patentinhaber: Otto Bock HealthCare GmbH, 37115 Duderstadt (DE)
(72) Erfinder: REINHARDT, Holger, 47906 Kempen (DE); KROLL-ORYWAHL, Olaf, 37083 Göttingen (DE)
(74) Vertreter: Stornebel, Kai
(86) Internationale Anmeldenummer: PCT/DE2010/001071
(87) Internationale Veröffentlichungsnummer: WO 2011/047647

(56) Entgegenhaltungen:
- WO-A1-98/42257
- WO-A1-2006/121413
- DE-A1-102005 058 850
- DE-U1-202008 003 075
- US-A- 5 143 088
- US-A- 5 259 833
- US-B1- 6 440 094

## Beschreibung

Die Erfindung betrifft eine Vorrichtung zur Erfassung und/oder Beeinflussung der Körperhaltung mit einem flexiblen Trägerelement, an dem eine Fixiereinrichtung zur Anordnung der Vorrichtung in oder unterhalb der Taillenhöhe eines Trägers der Vorrichtung angeordnet ist. Eine solche Vorrichtung dient insbesondere zur Bewegungsanalyse oder zur Unterstützung der Körperhaltung im Rumpfbereich.

Die DE 10 2005 058 850 A1 beschreibt einen Applikator zur selbsttätigen TENS-Behandlung am Körper mit einem hosenträgerartigen Gurtsystem, das am Bund einer Hose mit lösbaren Klemmen befestigt werden kann. An dem Gurtsystem sind verschiebbare Elektroden angebracht. Das Gurtsystem weist zwei Längsbänder auf, die über Distanzbänder nach innen gespannt und über Doppelspangen mit diesen verbunden sind.

Die US-A-5,143,088 beschreibt eine Vorrichtung zum Überwachen der Bewegungskomponenten der Wirbelsäule. Ein langgestrecktes Exoskelett mit einer Vielzahl im Wesentlichen flacher, T-förmiger Elemente, die in Längsrichtung zueinander beabstandet sind, ist an dem Rücken eines Menschen festgelegt. Die Festlegung erfolgt über einen Hüftgurt und seitlich vom oberen Ende des Exoskeletts abstehende Schultergurte. Die Vorrichtung wird über Klettverschlüsse an dem Körper befestigt.

Die DE 20 2008 003 075 U1 beschreibt ein elastisches Seilsystem zur Unterstützung der physiologischen Körperhaltung. Das elastische Seilsystem kann am Oberkörper eines Menschen angelegt werden und sieht ein flexibles Gummiseil vor, das durch zwei Federhaken zu einem Seilsystem verbunden und durch einen Kordelstopper in der Länge regulierbar ist. Eine Fehlstellung wird durch Druck im Bereich der Schlüsselbeine spürbar gemacht. Eine Fixierung im Bereich der Lendenwirbelsäule erfolgt über eine Gürtelschlaufe.

Die WO 2006/121413 A1 beschreibt eine Weste, die die Haltung verbessern soll. Ein Taillengürtel ist mit einem elastischen Rückenband versehen. An das elastische Rückenband sind im Schulterbereich elastische Streifen angeordnet, die an nicht elastischen Streifen oder Gurten befestigt sind. Die nicht elastischen Gurte sind wiederum an dem unteren Ende des Rückenbandes in der Länge einstellbar befestigt.

Die US 6 440 094 B1 beschreibt eine Vorrichtung zur Verbesserung der Körperhaltung. In einer Ausführungsform sind zwei Bänder im Rückenbereich eines Anwenders jeweils durch ein Schlauchelement einander kreuzend geführt, wobei jedes Band mit einem Ende an einem Schulterelement und einem anderen Ende an einem Taillengurt seitlich links bzw. rechts des Anwenders festlegbar ist. Diese Merkmale sind in ähnlicher Form in dem Oberbegriff des Anspruchs gewürdigt.

Aufgabe der vorliegenden Erfindung ist es, eine Vorrichtung bereitzustellen, die eine verbesserte Bewegungsanalyse ermöglicht und verbesserte Rückmeldungen über das Bewegungsverhalten liefert.

Erfindungsgemäß wird diese Aufgabe durch eine Vorrichtung mit den Merkmalen des Hauptanspruches gelöst. Vorteilhafte Ausgestaltungen und Weiterbildungen der Erfindung sind in den Unteransprüchen aufgeführt.

Die erfindungsgemäße Vorrichtung zur Erfassung und/oder Beeinflussung der Körperhaltung mit einem flexiblen Trägerelement, an dem eine Fixiereinrichtung zur Anordnung der Vorrichtung in oder unterhalb der Taillenhöhe angeordnet ist, wobei Spanngurte oder Zugelemente mit einem ersten Ende an der Fixiereinrichtung festgelegt und im Rückenbereich einander kreuzend geführt sind und mit einem zweiten Ende im frontalen Schulter- oder Brustbereich an dem Trägerelement festgelegt sind, sieht vor, dass zumindest ein Sensor dem Trägerelement, den Spanngurten und/oder den Versteifungselementen zugeordnet ist, der z.B. Kräfte, Verformungen, Winkel und/oder Beschleunigungen erfasst. Dadurch ist es möglich, die Vorrichtung mit einer Sensorik zu kombinieren, um verschiedene Bewegungszustände zu erfassen, zu dokumentieren und gegebenenfalls auszuwerten. Die Sensoren können beispielsweise als konduktive Textilfäden, Gyroskope, Beschleunigungsmesser, Dehnungsmesser, Druckmesser oder Temperatursensoren ausgebildet sein. Die Winkelmessung kann absolut oder relativ zu einer Komponente oder einem Körperteil erfolgen.

Insbesondere durch die gekreuzte Führung der Spanngurte ist es möglich, die grundsätzlich kritischen Torsionsbewegungen des Rumpfes zu erfassen und durch Ausübung einer Zugkraft oder Druckkraft auf der jeweils nach vorne verdrehten Seite eine Rückmeldung zu geben, dass sich gegenwärtig der Rumpf in einer tordierten Stellung befindet. Sofern sich der Träger der Vorrichtung gleichmäßig nach vorne beugt, wird durch die Ausübung von Zug und Druck im Schulter- oder Brustbereich die gleichmäßige Beugung angezeigt, so dass der Träger der Vorrichtung zu einer aufrechten Körperhaltung angehalten wird oder ihm zumindest verdeutlicht wird, in welcher Position und Haltung er sich befindet.

An dem Trägerelement können im frontalen Schulter- oder Brustbereich Befestigungseinrichtungen angeordnet sein, an denen die zweiten Enden der Spanngurte reversibel festlegbar sind. Dadurch ist es möglich, dass die ausgeübte Gegenkraft durch die Spanngurte stufenlos einstellbar ist. Die jeweils gewünschte Haltung kann dadurch stufenlos eingestellt werden, so dass eine Anpassung an die jeweils gewünschte Körperhaltung leicht möglich ist. Die Anordnung der Befestigungseinrichtungen im Brust- oder Schulterbereich erleichtert die Einstellung, da diese Körperregionen leicht zu erreichen sind. Die ersten Enden, die an der Fixiereinrichtung im Taillenbereich festgelegt sind, sind bevorzugt permanent festgelegt, beispielsweise durch Vernähen oder Verschweißen, alternativ können auch diese Enden an der Fixiereinrichtung im Taillenbereich reversibel festlegbar sein, beispielsweise über Haken-Flausch-Verschlüsse oder andere formschlüssige Verbindungen wie Schnallen, Knöpfe oder dergleichen.

Bevorzugt ist das Trägerelement als eine Jacke oder Weste ausgebildet, die leicht anlegbar sind. Die Jacke oder Weste kann im vorderen Bereich geöffnet werden, beispielsweise über einen Reißverschluss oder über einen Flausch-Haken-Verschluss und ebenso einfach wieder geschlossen werden, so dass das Trägerelement fest an dem Körper anliegt. Im Rückenbereich kann das Trägerelement vollflächig ausgebildet sein und den Rücken vollständig überdecken, es ist aber auch möglich, dass das Trägerelement Durchbrechungen aufweist, so dass der Rückenbereich und der Frontalbereich nur teilweise von dem Trägerelement abgedeckt sind. Das Trägerelement kann dabei unmittelbar auf der Haut über entsprechenden Kleidungsstücken getragen werden.

Die Fixiereinrichtung zum Festlegen der Vorrichtung im Bereich der Taillenhöhe oder unterhalb davon ist bevorzugt als ein Spanngurt ausgebildet, der Teil des Trägerelementes sein kann. Der Spanngurt oder Taillengurt kann an dem übrigen Teil des Trägerelementes, beispielsweise am unteren Ende der Weste oder der Jacke befestigt oder daran ausgebildet sein, um einen integralen Bestandteil der Trägereinrichtung auszubilden. Auf diese Art und Weise wird das Anlegen der Vorrichtung vereinfacht, da lediglich die Jacke oder Weste angelegt und der Spanngurt gespannt werden muss, um eine ausreichende Umfangskraft auszuüben, damit die Fixiereinrichtung an dem Körper sicher gehalten wird. Alternativ dazu ist es vorgesehen, dass die Fixiereinrichtung als separate Komponente ausgebildet sind, die miteinander gekoppelt werden können, so dass beispielsweise das Anlegen der Fixiereinrichtung unabhängig von dem Anlegen des Trägerelementes erfolgen kann.

Neben der Ausgestaltung der Fixiereinrichtung als Spanngurt, der um den Rumpf herum angelegt wird, ist es möglich, dass diese als eine Hose oder eine Gurtung im Schritt ausgebildet ist. Dabei ist es das Ziel der Fixiereinrichtung, dass das Trägerelement distal festgelegt wird, um ein Hochrutschen zu verhindern.

In dem Trägerelement oder der Fixiereinrichtung kann eine Lordosenstütze angeordnet sein, um die Körperhaltung weiter zu unterstützen. Diese Lordosenstütze kann reversibel an dem Trägerelement und/oder der Fixiereinrichtung angeordnet sein, beispielsweise in Gestalt einer Tasche, in die ein Stützelement einführbar ist. Ebenfalls ist es möglich, dass die Lordosenstütze auf der Innen- oder Außenseite des Trägerelementes oder der Fixiereinrichtung an der Vorrichtung befestigt wird.

In dem Trägerelement, der Fixiereinrichtung und/oder den Spanngurten können Versteifungselemente angeordnet sein, um für den Träger der Vorrichtung eine zusätzliche Stabilität bereitzustellen. Die Versteifungselemente können sowohl in dem Trägerelement als auch in der Fixiereinrichtung und/der den Spanngurten angeordnet sein oder an diesen befestigt sein. In einer Variante der Erfindung ist es vorgesehen, dass Taschen zur Aufnahme von Versteifungselementen in dem Trägerelement bzw. der Fixiereinrichtung und/oder den Spanngurten ausgebildet sind, so dass die Versteifungselemente leicht in diese Taschen eingeschoben und aus diesen entfernt werden können, wenn die Versteifungselemente nicht mehr benötigt werden.

An dem Trägerelement können Haftelemente angeordnet sein, um ein Verrutschen des Trägerelementes auf der Haut oder auf der Kleidung zu vermeiden oder zu minimieren. Die Haftelemente können punktuell oder großflächig auf der Innenseite des Trägerelementes und/oder der Fixiereinrichtung angeordnet sein. Je größer die Haftelemente sind, desto größer ist die Fixierwirkung und desto geringer ist die Relativbewegung zwischen dem Trägerelement und dem Körper bzw. der Kleidung.

Es kann weiterhin vorgesehen sein, dass ein Datenlogger oder eine Datenübermittlungsschnittstelle mit dem Sensor oder den Sensoren verbunden ist, um die Daten aufzuzeichnen oder weiterzuleiten. Ebenfalls ist es möglich, dass nach Auswertung der Daten Warnsignale ausgegeben werden, wenn bestimmte Parameter überschritten werden. Ebenfalls ist es möglich, die Sensoren in einem Biofeedbackverfahren einzusetzen, um bei der Ausführung bestimmter Bewegungen bestimmte Rückmeldungen zu erhalten, beispielsweise Vibrationen, akustische Signale oder dergleichen. Die Vorrichtung ist auch bei der funktionalen Elektrostimulation einsetzbar, um gezielt Bewegungen aufzubauen oder zu kontrollieren.

Die Spanngurte sind bevorzugt flexibel und elastisch, wobei eine Dehnungsbegrenzung in den Spanngurten vorgesehen sein kann, so dass nach Erreichen einer bestimmten Haltung oder Erreichen oder Überschreiten einer Dehnung der Spanngurte diese unelastisch werden, wodurch sich eine spürbare Veränderung der Gegenkraft im Bereich der Schultern oder des Brustkorbes ergibt. Die Elastizität ist sowohl in Zugrichtung als auch in Biegerichtung gegeben, so dass die Spanngurte zug- und biegeelastisch ausgebildet sein können.

Das Trägerelement besteht vorzugsweise aus einem scherstabilen Material, so dass keine Relativbewegung zwischen der Oberseite und der Unterseite des Trägerelementes bewirkt wird. Die Oberseite ist dem Körper des Trägers der Vorrichtung abgewandt, während die Unterseite dem Körper des Trägers zugewandt ist und auf der Haut oder der Kleidung anliegt.

Nachfolgend wird ein Ausführungsbeispiel der Erfindung anhand der beigefügten Figuren näher erläutert. Es zeigen:
- Figur 1: eine angelegte Vorrichtung in Frontalansicht;
- Figur 2: eine angelegte Vorrichtung in Rückenansicht; sowie
- Figur 3: eine angelegte Vorrichtung in perspektivischer Darstellung.

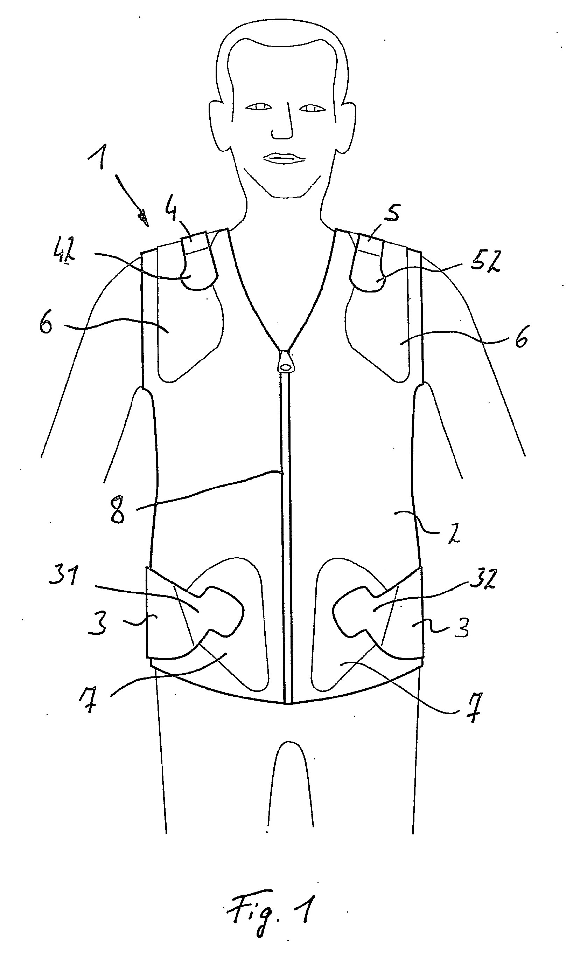

In der Figur 1 ist in einer Frontalansicht die Vorrichtung 1 zur Erfassung und/oder Beeinflussung der Körperhaltung gezeigt, die ein flexibles Trägerelement 2 aufweist, das in der dargestellten Ausführungsform in Gestalt einer Weste ausgebildet ist. Das Trägerelement 2 kann aus einem textilen Material bestehen, das vorzugsweise innenseitig mit einer rutschfesten oder rutschhemmenden Beschichtung versehen ist, um das Trägerelement sicher an dem Körper des Trägers der Vorrichtung 1 zu halten. Das Trägerelement 2 kann neben einer Flexibilität auch eine Elastizität aufweisen, um ein möglichst enges Anliegen an dem Körper zu ermöglichen. Die Elastizität kann auch nur bereichsweise ausgebildet sein, um die Passform zu verbessern, wohingegen die flexiblen und unelastischen Bereiche zu einer unmittelbaren Kraftübertragung und Kraftweiterleitung ausgebildet sein können. Das Trägerelement 2 weist im vorderen Bereich einen Reißverschluss 8 auf, über den das Trägerelement 2 nach dem Anlegen geschlossen werden kann, um eine verbesserte Passform und einen besseren Sitz des Trägerelementes 2 an dem Körper zu bewirken. Statt eines Reißverschlusses 8 können andere Verschlusseinrichtungen vorgesehen sein, beispielsweise ein Haken-Flausch-Verschluss, ein sogenannter Klettverschluss, oder andere Verbindungs- oder Fixiereinrichtungen.

Im Bereich der Taille oder der Hüfte ist eine Fixiereinrichtung 3 an dem Trägerelement 2 angeordnet, beispielsweise daran ausgebildet oder befestigt. Die Fixiereinrichtung 3 ist als ein Spanngurt vorgesehen, der über den rückwärtigen Taillenbereich geschlossen ausgebildet ist und im Frontalbereich zwei Enden 31, 32 aufweist, die relativ zueinander verlagerbar sind. Werden die Enden 31, 32 aufeinander zu bewegt, wird die Fixiereinrichtung 3 gespannt, wodurch eine größere Haltekraft auf den Taillenbereich ausgeübt wird. Die Verlagerung und Fixierung der Enden 31, 32 erfolgt im dargestellten Ausführungsbeispiel über Befestigungsbereiche 7, die als Teil eines Haken-Flausch-Verschlusses ausgebildet sind. Sind an den Enden 31, 32 der Fixiereinrichtung 3 innenseitig Hakenbereiche vorgesehen, sind die Befestigungsbereiche 7 als sogenannte Flauschbereiche ausgebildet, die sich im dargestellten Ausführungsbeispiel beiderseits des Reißverschlusses 7 von dem unteren Rand des Trägerelementes 2 bis ungefähr in Höhe des Bauchnabels erstrecken. Durch die Ausgestaltung der Befestigungsbereiche 7 und der Enden 31, 32 als Klettverschlussverbindungen, ist eine stufenlose Anpassung der Fixiereinrichtung 3 an den jeweiligen Träger möglich.

Im Schulter- und oberen Brustbereich des Trägerelements 2 sind Befestigungseinrichtungen 6 angeordnet, an denen Zugelemente oder Spanngurte 4, 5 festgelegt werden können. Korrespondierend zu der Fixiereinrichtung 3 der Befestigungsbereiche 7 sind die Befestigungseinrichtungen 6 und die Enden 42, 52 der Spanngurte 4, 5 als Teile eines Klettverschlusses ausgebildet, die Befestigungseinrichtungen 6 sind vorzugsweise als Flauschbereiche, die Enden 42, 52 der einzelnen Spanngurte 4, 5 als Hakenbereiche vorgesehen. Die Positionierung der Enden 42, 52 der Spanngurte 4, 5 kann an den Befestigungseinrichtungen 6 frei gewählt werden, so dass die Feinausrichtung der Enden 42, 52 und die Spannung der Spanngurte 4, 5 individuell einstellbar ist.

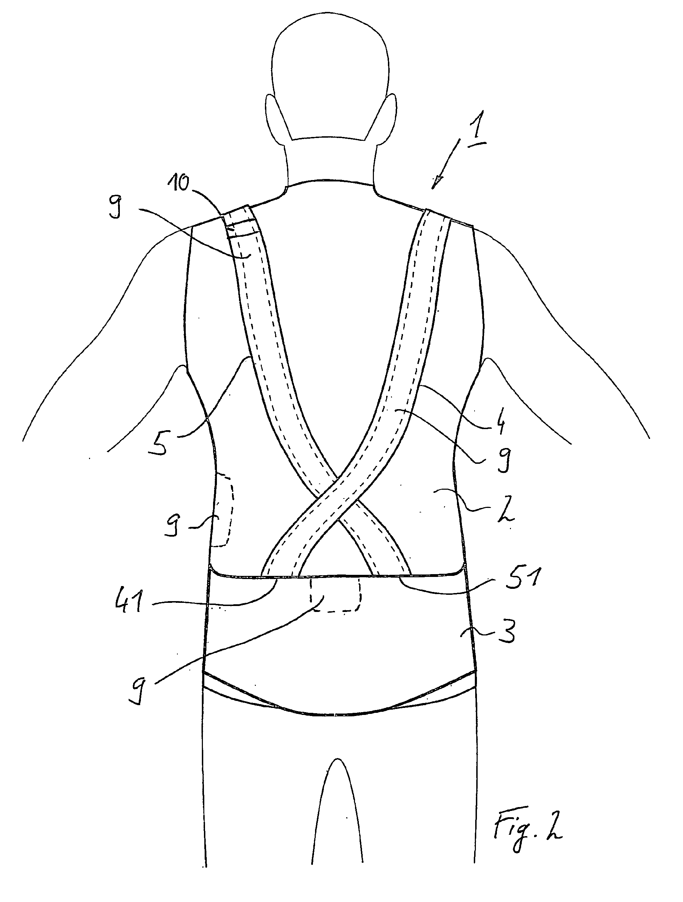

In der Figur 2 ist eine Rückenansicht der Vorrichtung 1 gezeigt. In der Figur 2 ist zu erkennen, dass die Fixiereinrichtung 3 im Hüft- und Taillenbereich angeordnet ist und im Dorsalbereich durchgehend ausgebildet ist. Nach oben schließt sich das Material des Trägerelementes 2 an. Weiterhin ist zu erkennen, dass die Spanngurte 4, 5 im Rückenbereich, ungefähr auf Höhe der oberen Lendenwirbel einander kreuzend ausgeführt sind, wobei die ersten Enden 41, 51 der Spanngurte 4, 5 im Bereich der Fixiereinrichtung 3 festgelegt sind. Die Festlegung kann entweder permanent durch Annähen oder Anschweißen oder reversibel durch Befestigungseinrichtungen wie Klettverschluss, Schnallen, Gurtschlaufen oder Einknöpfungen erfolgen. Die ersten Enden 41, 51 der Spanngurte 4, 5 sind beidseitig der Wirbelsäule, also beidseitig neben der Mittellinie des Trägerelementes 2 angeordnet. Der Abstand der ersten Enden 41, 51 von der Mittellinie kann relativ frei gewählt werden, je größer der Abstand ist, desto größer ist die Auswirkung einer Torsionsbewegung auf die zweiten Enden 42, 52.

Ausgehend von den Befestigungsstellen der ersten Enden 41, 51 werden die Spanngurte 4, 5 zunächst nach oben in Richtung der Schulterbereiche geführt und im dargestellten Ausführungsbeispiel im Lendenwirbelbereich überkreuzt. Im weiteren Verlauf erstrecken sich die Spanngurte 4, 5 zu den den Befestigungsstellen schräg gegenüberliegenden Schultern und werden entweder im oberen Schulterbereich oder im frontalen Brustbereich an den Befestigungseinrichtungen festgelegt. Die Spanngurte 4, 5 können über die gesamte Länge elastisch ausgebildet oder auch nur über ihre Länge teilweise elastisch ausgebildet sein. Beispielsweise kann von den ersten Befestigungsstellen der ersten Enden 41, 51 eine vorbestimmte Länge der jeweiligen Spanngurte 4, 5 flexibel und unelastisch ausgebildet sein, während ein sich daran anschließender Bereich, beispielsweise im Bereich der Schultern, elastisch ausgebildet ist. Ebenfalls kann eine Dehnungsbegrenzung in den Spanngurten 4, 5 angeordnet sein, die nach Erreichen einer Maximaldehnung wirksam wird, so dass nach Erreichen einer vorgegebenen Dehnung keine zusätzliche Längung der Spanngurte 4, 5 erfolgen kann.

Spanngurte 4, 5 können durchgängig aus einem einzigen Material ausgebildet sein. Ebenfalls können die Spanngurte 4, 5 unterschiedliche Materialien aufweisen und beispielsweise abschnittsweise aus unterschiedlichen Materialien aufgebaut sein. Die Spanngurte 4, 5 können als Gewebegurte, Seile, Kabel, Kabelzüge oder dergleichen ausgebildet sein. Durch die Überkreuzung der Spanngurte oder Zugelemente 4, 5 ergibt sich ein Kräftekoordinatensystem, das insbesondere Torsionsbewegungen des Trägers der Vorrichtung erfassbar und spürbar macht.

In der Fixiereinrichtung 3 ebenso wie in dem Trägerelement 2 können Taschen oder Aufnahmeeinrichtungen 9 für Versteifungselemente oder Stützelemente ausgebildet sein. Im Bereich der zentralen Fixiereinrichtung 3 kann eine Lordosenstütze in eine Tasche 9 eingeführt werden, im Taillenbereich können Taschen 9 für Versteifungselemente in dem Trägerelement 2 angeordnet oder ausgebildet sein, so dass auf einfache Art und Weise Polster, Versteifungen, Stützeinrichtungen oder dergleichen einsteckbar und wieder entnehmbar sind. Statt Taschen 9 können auch andere Aufnahmeeinrichtungen vorgesehen sein, an denen die Versteifungselemente festgelegt oder in die die Versteifungselemente oder Stützelemente eingeführt werden können.

In dem dargestellten Ausführungsbeispiel ist in dem über die linke Schulter geführten Spanngurt 5 ein Sensor 10 vorgesehen, der beispielsweise als Zugkraftsensor ausgebildet sein kann. Bei Überschreiten eines vorgegebenen Sensorwertes können Warnsignale ausgegeben werden, um zu verhindern, dass der Träger der Vorrichtung 1 ungünstige Bewegungen ausführt. Neben Zugkraftsensoren können Sensoren für Beschleunigungen, Dehnungen, Druck, Temperatur oder dergleichen vorgesehen sein. Ebenfalls können konduktive Textilfäden vorgesehen sein, über die Sensorsignale abgeleitet werden. Die Textilfäden können in den Gurten 4, 5 oder in dem Material des Trägerelementes 2 angeordnet sein. Die Sensorsignale können in einem Biofeedbackverfahren mit Vibrationsgebern oder Schallerzeugern oder Elektrostimulationsgeräten gekoppelt werden, um eine Rückmeldung über die gerade vorliegende Haltung oder Bewegung geben zu können. Ebenfalls können Datenspeicher oder Datenübermittlungseinrichtungen vorgesehen sein, um die Sensordaten zu speichern oder an eine Auswerteeinrichtung zu übermitteln.

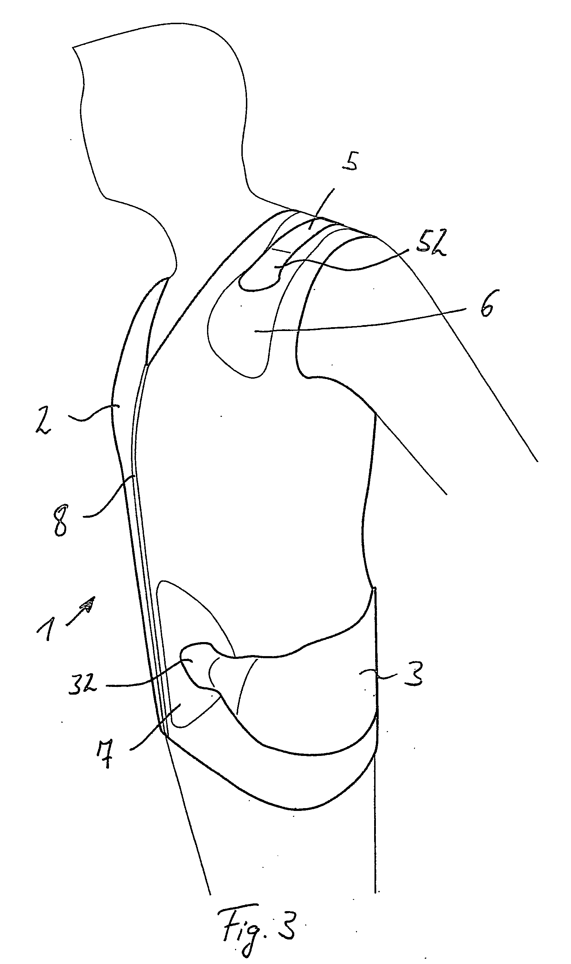

Die Fixiereinrichtung 3 ist so angeordnet und ausgebildet, dass die Umfangskraft, die durch die Fixiereinrichtung 3 ausgeübt wird, größer als die Zugkraft der Spanngurte 4, 5 ist, um eine Verlagerung des Trägerelementes 2 relativ zu dem Träger zu verhindern. In der Figur 3 ist eine perspektivische Ansicht der Vorrichtung 1 dargestellt, in der zu erkennen ist, dass die Fixiereinrichtung 3 ein offenes Ende 32 aufweist, das an den Befestigungsbereichen 7 reversibel und stufenlos festlegbar ist. Das zweite Ende 52 des Spanngurtes 5 ist in Blickrichtung gesehen im linken Schulterbereich bzw. im vorderen linken Brustbereich des Trägers festgelegt, während das erste Ende 51 in Blickrichtung gesehen rechts neben der Mittellinie des Trägerelementes 2 und damit rechts neben der Wirbelsäule des Trägers angeordnet ist.

Sensoren 10 können auch in Versteifungselementen angeordnet sein und mit Sende- und/oder Speichereinrichtungen versehen sein, um die Bewegungen oder Belastungen zu dokumentieren oder die entsprechenden Daten weiterzuleiten.

Durch die reversible Befestigbarkeit der flexiblen Spanngurte oder Zugeinrichtungen 4, 5 ist es möglich, die Spannung der Spanngurte 4, 5 einzustellen und Torsionsbewegungen über Kräfte im Bereich der Schultern oder im vorderen Brustbereich bemerkbar zu machen. Ebenfalls ist es möglich, eine Gegenkraft gegen die Torsions- oder Beugebewegung bereitzustellen, um eine Verdrehung des Rumpfes oder eine Krümmung der Wirbelsäule zu verhindern, wenn dies gewünscht wird.

## Patentansprüche

1. Vorrichtung zur Erfassung und/oder Beeinflussung der Körperhaltung mit einem flexiblen Trägerelement (2), an dem eine Fixiereinrichtung (3) zur Anordnung der Vorrichtung (1) in oder unterhalb der Taillenhöhe angeordnet ist, wobei Spanngurte (4, 5) mit einem ersten Ende (41, 51) an der Fixiereinrichtung (3) festgelegt und im Rückenbereich einander kreuzend geführt sind und mit einem zweiten Ende (42, 52) im frontalen Schulter- oder Brustbereich an dem Trägerelement (2) festgelegt sind **dadurch gekennzeichnet, dass** zumindest ein Sensor (10) dem Trägerelement (2), den Spanngurten (4, 5) und/oder Versteifungselementen zugeordnet ist, der Kräfte, Verformungen, Winkel, Temperaturen und/oder Beschleunigungen erfasst.

2. Vorrichtung nach Anspruch 1, **dadurch gekennzeichnet, dass** an dem Trägerelement (2) im frontalen Schulter- oder Brustbereich Befestigungseinrichtungen (6) angeordnet sind, an denen die zweiten Enden (42, 52) der Spanngurte (4, 5) reversibel festlegbar sind.

3. Vorrichtung nach Anspruch 1 oder 2, **dadurch gekennzeichnet, dass** das Trägerelement (2) als Jacke oder Weste ausgebildet ist.

4. Vorrichtung nach einem der voranstehenden Ansprüche, **dadurch gekennzeichnet, dass** die Fixiereinrichtung (3) als Spanngurt ausgebildet ist, der Teil des Trägerelementes (2) ist.

5. Vorrichtung nach einem der Ansprüche 1 bis 3, **dadurch gekennzeichnet, dass** die Fixiereinrichtung (3) und das Trägerelement (2) separat ausgebildet sind.

6. Vorrichtung nach einem der voranstehenden Ansprüche, **dadurch gekennzeichnet, dass** die Fixiereinrichtung (3) als Hose oder Gurtung im Schritt ausgebildet ist.

7. Vorrichtung nach einem der voranstehenden Ansprüche, **dadurch gekennzeichnet, dass** in dem Trägerelement (2) oder in der Fixiereinrichtung (3) eine Lordosenstütze angeordnet ist.

8. Vorrichtung nach einem der voranstehenden Ansprüche, **dadurch gekennzeichnet, dass** in dem Trägerelement (2) Versteifungselemente angeordnet sind.

9. Vorrichtung nach einem der voranstehenden Ansprüche, **dadurch gekennzeichnet, dass** in den Trägerelement (2) und/oder der Fixiereinrichtung (3) und/oder in den Spanngurten (4, 5) Taschen (9) zur Aufnahme von Versteifungs- oder Stützelementen ausgebildet sind.

10. Vorrichtung nach einem der voranstehenden Ansprüche, **dadurch gekennzeichnet, dass** an dem Trägerelement (2) innenseitig Haftelemente angeordnet sind.

11. Vorrichtung nach Anspruch 1, **dadurch gekennzeichnet, dass** ein Datenlogger oder eine Datenübermittlungsschnittstelle mit dem Sensor (10) verbunden ist.

12. Vorrichtung nach einem der voranstehenden Ansprüche, **dadurch gekennzeichnet, dass** die Spanngurte (4, 5) flexibel und elastisch sind.

13. Vorrichtung nach einem der voranstehenden Ansprüche, **dadurch gekennzeichnet, dass** das Trägerelement (2) aus einem scherstabilen Material ausgebildet ist.

## Claims

1. device for detecting and/or influencing posture, having a flexible support element (2), on which a fixing apparatus (3) is arranged for arranging the device (1) at waist height or below, with tension straps (4, 5) being fixed on the fixing apparatus (3) with a first end (41, 51) and guided so as to cross each other in the region of the back and being fixed to the support element (2) in the frontal shoulder or chest region with a second end (42, 52), **characterized in that** at least one sensor (10) is associated with the support element (2), the tension straps (4, 5) and/or stiffening elements and it detects forces, deformations, angles, temperatures and/or accelerations.

2. The device as claimed in claim 1, **characterized in that** attachment apparatuses (6) are arranged on the support element (2) in the frontal shoulder or chest region and the second ends (42, 52) of the tension straps (4, 5) can be reversibly fixed to these.

3. The device as claimed in claim 1 or 2, **characterized in that** the support element (2) is embodied as a jacket or vest.

4. The device as claimed in one of the preceding claims, **characterized in that** the fixing apparatus (3) is embodied as a tension strap which is part of the support element (2).

5. The device as claimed in one of claims 1 to 3, **characterized in that** the fixing apparatus (3) and the support element (2) are embodied separately.

6. The device as claimed in one of the preceding claims, **characterized in that** the fixing apparatus (3) is embodied as a pair of trousers or as a strap in the crotch.

7. The device as claimed in one of the preceding claims, **characterized in that** a lumbar support is arranged in the support element (2) or in the fixing apparatus (3).

8. The device as claimed in one of the preceding claims, **characterized in that** stiffening elements are arranged in the support element (2).

9. The device as claimed in one of the preceding claims, **characterized in that** pockets (9) for holding stiffening or supporting elements are formed in the support element (2) and/or the fixing apparatus (3) and/or in the tension straps (4, 5).

10. The device as claimed in one of the preceding claims, **characterized in that** adhesion elements are arranged on the inside of the support element (2).

11. The device as claimed in claim 11, **characterized in that** a data logger or a data transmission interface is connected to the sensor (10).

12. The device as claimed in one of the preceding claims, **characterized in that** the tension straps (4, 5) are flexible and elastic.

13. The device as claimed in one of the preceding claims, **characterized in that** the support element (2) is made of a shear-stable material.

## Revendications

1. Dispositif pour appréhender et/ou influencer la posture corporelle, comprenant un élément porteur flexible (2) sur lequel est agencé un système de fixation (3) pour agencer le dispositif (1) à la hauteur de la taille ou au-dessous de celle-ci, dans lequel des sangles de serrage (4, 5) sont immobilisées par une première extrémité (41, 51) sur le système de fixation (3) et sont guidées en se croisant mutuellement dans la région dorsale, et sont immobilisées par une seconde extrémité (42, 52) sur l'élément porteur (2) dans la région des épaules ou du thorax,
**caractérisé en ce qu'**au moins un capteur (10) est associé à l'élément porteur (2), aux sangles de serrage (4, 5) et/ou à des éléments de rigidification, ce capteur détectant des forces, des déformations, des angles, des températures et/ou des accélérations.

2. Dispositif selon la revendication 1, **caractérisé en ce que** des systèmes de fixation (6) sont agencés sur l'élément porteur (2) dans la région des épaules ou du thorax, sur lesquels les secondes extrémités (42, 52) des sangles de serrage (4, 5) peuvent être immobilisées de façon réversible.

3. Dispositif selon la revendication 1 ou 2, **caractérisé en ce que** l'élément porteur (2) est réalisé comme une veste ou un gilet.

4. Dispositif selon l'une des revendications précédentes, **caractérisé en ce que** le système de fixation (3) est réalisé comme une sangle de serrage qui fait partie de l'élément porteur (2).

5. Dispositif selon l'une des revendications 1 à 3, **caractérisé en ce que** le système de fixation (3) et l'élément porteur (2) sont réalisés séparément.

6. Dispositif selon l'une des revendications précédentes, **caractérisé en ce que** le système de fixation (3) est réalisé comme une culotte ou comme un harnais d'entrejambe,

7. Dispositif selon l'une des revendications précédentes, **caractérisé en ce qu'**un soutien lombaire est agencé dans l'élément porteur (2) ou dans le système de fixation (3).

8. Dispositif selon l'une des revendications précédentes, **caractérisé en ce que** des éléments de rigidification sont agencés dans l'élément porteur (2).

9. Dispositif selon l'une des revendications précédentes, **caractérisé en ce que** des poches (9) destinées à recevoir des éléments de rigidification ou de soutien sont réalisées dans l'élément porteur (2) et/ou dans le système de fixation (3) et/ou dans les sangles de serrage (4, 5).

10. Dispositif selon l'une des revendications précédentes, **caractérisé en ce que** des éléments adhésifs sont agencés du côté intérieur sur l'élément porteur (2).

11. Dispositif selon la revendication 1, **caractérisé en ce qu'**un collecteur de données ou une interface de transmission de données est connectée au capteur (10)

12. Dispositif selon l'une des revendications précédentes, **caractérisé en ce que** les sangles de serrage (4, 5) sont flexibles et élastiques.

13. Dispositif selon l'une des revendications précédentes, **caractérisé en ce que** l'élément porteur (2) est réalisé en un matériau stable vis-à-vis du cisaillement.
